# EUROPEAN PATENT SPECIFICATION

(11) **EP 3 995 744 B1**
(45) Date of publication and mention of the grant of the patent: **11.10.2023**
(21) Application number: 21848122.4
(22) Date of filing: 23.08.2021
(51) Int. Cl.: F24C 15/20, A61L 9/014, A61L 9/14, A61L 9/20

(54) **RECIRCULATION HOOD WITH AIR COOLING UNIT**
UMLUFTHAUBE MIT LUFTKÜHLEINHEIT
HOTTE DE RECIRCULATION AVEC UNITÉ DE REFROIDISSEMENT D' AIR

(30) Priority: 27.08.2020 CN 202010876691
(43) Date of publication of application: 11.05.2022
(73) Proprietor: SHANGHAI DUOHUAN OIL-SMOKE PURIFICATION EQUIPMENT CO., LTD., Shanghai 201801 (CN)
(72) Inventor: HE, Yang, Shanghai 201801 (CN); HE, Weibin, Shanghai 201801 (CN)
(74) Representative: Patentship Patentanwaltsgesellschaft mbH
(86) International application number: PCT/CN2021/113985
(87) International publication number: WO 2022/042462

(56) References cited:
- CN-A- 103 353 138
- CN-A- 103 353 138
- CN-A- 107 327 889
- CN-A- 108 426 285
- CN-A- 109 028 189
- CN-A- 111 895 470
- CN-A- 111 921 323
- CN-A- 113 188 173
- CN-U- 2 114 109
- DE-A1- 1 642 116
- DE-A1-102007 042 284
- GB-A- 2 262 895
- US-A- 4 050 446

## Description

### TECHNICAL FIELD

The present disclosure relates to a lampblack purifier, in particular to an internal circulation type lampblack purifier used indoors.

### BACKGROUND ART

People expect to breathe fresh air when haze is shaded and viruses are raging. According to scientific analysis, harmful gas emitted by catering (including hotel catering and home catering) is one of important factors influencing atmospheric pollution. In order to control the emission of catering to the atmosphere, although strict regulations and measures are continuously adopted at the policy level, the degree of atmospheric pollution cannot be fundamentally improved due to the insufficiency of technical means.

In order to meet the requirements of public social environment and life of people, a circulation type range hood or an internal circulation type lampblack purifier is provided in the prior art, and lampblack, peculiar smell and harmful gas generated in cooking are directly purified indoors, and clean gas generated after purification returns to the room again and is not discharged to the outside. Internal circulation type lampblack purifiers are already applied in western developed countries, and some countries even adopt legal bans to prohibit direct discharge of catering to the outside. Although many existing patents basically realize pollution-free emission of catering cooking to the outside, a plurality of defects still exist in the purification effect, particularly, although gas is purified in the purification process, the temperature of the discharged gas is high, the gas is not effectively cooled, and when the gas returns to the room again, the rise of indoor temperature and overflow of a large amount of water vapor are caused, so that discomfort of cooking operators is caused and is particularly prominent in hot summer.

DE 1642116 A1 discloses a vapor extractor, in which the air to be cleaned is sucked by means of a blower through a filter and past an ozone lamp and fed back into the room, characterized by an air washer (26) downstream of the ozone lamp (20).

CN 108426285 A relates to the technical field of environmental protection, in particular to an environment-friendly range hood. The range hood comprises a smoke suction box, wherein a support rod is fixedly connected with an inner side of an upper end face of the smoke suction box; a fan is fixedly connected onto the support rod; a driving bevel gear is fixedly connected with a spindle of the fan; a first rotating rod and a second rotating rod which are arranged sequentially from top to bottom are rotationally connected with the inner side of the smoke suction box; the second rotating rod is fixedly connected with second gears; hair brushes are connected with an underneath of the second rotating rod in a sliding manner; a smoke suction pipe is communicated with an upside of the smoke suction box; an oil smoke treatment box is communicated with an upside of the smoke suction pipe; spray heads are fixedly connected in the oil smoke treatment box; and a condenser is fixedly connected with an upper end face in the oil smoke treatment box.

### SUMMARY

Aiming at the defects and disadvantages in the prior art, the present disclosure provides an internal circulation type lampblack purifier as defined in the independent claim 1, which can purify lampblack, peculiar smell and harmful gas generated by catering cooking, effectively reduce the temperature of exhausted gas and eliminate the influence of water vapor on the indoor environment. Further advantaged embodiments are defined in the dependent claims.

In order to achieve the purpose, the present disclosure aims to provide an internal circulation type lampblack purifier, comprising a shell, an air inlet, an air outlet, an air inlet channel, an air outlet channel, a fan device, a mechanical grease separator, a gas purification device and a cooling device, wherein the air inlet and the air outlet are arranged on the shell; one end of the air inlet channel communicates with the air inlet, the other end of the air inlet channel communicates with the fan device; one end of the air outlet channel communicates with the air outlet, and the other end of the air outlet channel communicates with the fan device; the mechanical grease separator is arranged at the air inlet or in the air inlet channel; and the cooling device is arranged at the air outlet and comprises a water supply pipe, water spray guide plates, a water mist baffle plate, a spray pipe and a water receiving tank, the spray pipe communicates with the water supply pipe, spray nozzles are arranged on the spray pipe and face the water spray guide plates, the water receiving tank is located below the water spray guide plates.

The water spray guide plate is of a transparent honeycomb net structure.

The water mist baffle plate is provided with blades at certain gaps, and the blades are folded or bent inwards.

The cooling device further comprises a water storage tank and a water pump, and the water supply pipe is connected with the water storage tank. Further, the water storage tank and the water receiving tank are combined into a whole.

The cooling device further comprises a drainage pipe, one end of the drainage pipe communicates with the water receiving tank, and the other end of the drainage pipe communicates with the outside.

The mechanical grease separator is of a double-layer concave-convex oppositely buckled grease separation screen plate structure or a motor-driven radiation type centrifugal screen disc structure.

The gas purification device is one or different combinations of more than two of an electrostatic lampblack purifier, an HEPA (High Efficiency Particulate Air) high-efficiency filter screen, an activated carbon filter screen, a TiO2 photocatalyst filter screen and an ultraviolet lamp.

According to the internal circulation type lampblack purifier, the cooling device is arranged, the temperature of exhausted gas is effectively reduced, high-temperature water vapor in the exhausted gas is eliminated, the influence of the high-temperature water vapor on the indoor environment is avoided, the exhausted gas cannot generate obvious influence on the indoor temperature, the situation that indoor personnel feel uncomfortable is avoided, and meanwhile, due to the fact that the mechanical grease separator and the gas purification device are arranged, a large amount of harmful pollution gas generated in catering cooking is purified, the internal circulation type lampblack purifier is a range hood and also is an air purifier, zero emission to the outside and no pollution to the inside are achieved, the atmospheric environment can be improved, and people can obtain a good cooking operation environment.

The concept, specific structure and resulting technical effects of the present disclosure will be further described below in conjunction with the attached figures so as to fully understand the objects, features and effects of the present disclosure.

### BRIEF DESCRIPTION OF THE DRAWINGS

FIG. 1 is a section view when an internal circulation type lampblack purifier is separated from a water mist baffle plate in the present disclosure;
FIG. 2 is a schematic diagram of a cooling device; and
FIG. 3 is a side profile of a cooling device.

### DETAILED DESCRIPTION OF THE EMBODIMENTS

The present disclosure is further described below in combination with the attached figures.

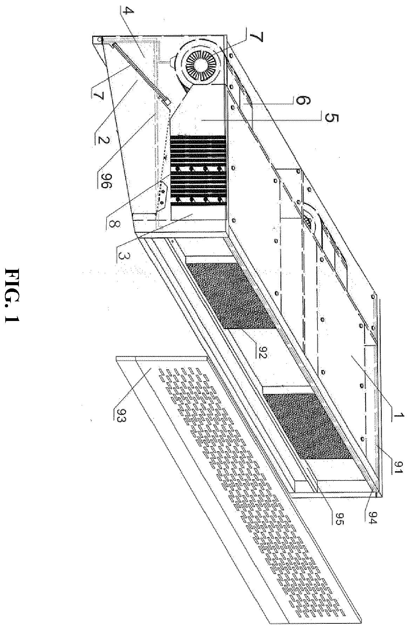

As shown in FIG. 1, an internal circulation type lampblack purifier in the present disclosure comprises a shell 1, an air inlet 2, an air outlet 3, an air inlet channel 4, an air outlet channel 5, a fan device 6, a mechanical grease separator 7, a gas purification device 8 and a cooling device 9, wherein the air inlet 2 and the air outlet 3 are arranged on the shell 1; the air inlet 2 faces the surface of a cooking bench and faces the interior; one end of the air inlet channel 4 communicates with the air inlet 2, the other end of the air inlet channel 4 communicates with the fan device 6; one end of the air outlet channel 5 communicates with the air outlet 3, and the other end of the air outlet channel 5 communicates with the fan device 6, namely, the air inlet channel 4 communicates with the air outlet channel 5 through the fan device 6; the mechanical grease separator 7 is arranged at the air inlet 2 or in the air inlet channel 4; and the cooling device 9 is arranged at the air outlet 3.

The mechanical grease separator 7 is of a double-layer concave-convex oppositely buckled grease separation screen plate structure or a motor-driven radiation type centrifugal screen disc structure. It is worthy remembering that the two structures can be independently used, and can also be used at the same time. The function of the mechanical grease separator 7 is that when mixed gas such as lampblack, peculiar smell and harmful gas generated in cooking is sucked and discharged by the fan device 6, grease and other gases in the mixed gas are effectively separated through the mechanical grease separator 7, the separated grease is collected through flow guiding, and other gases enter the air outlet channel 5 through the fan device 6, so that grease in the mixed gas is effectively separated from the other gases. Preferably, when the mechanical grease separator 7 is of a grease separation screen plate structure, the mechanical grease separator 7 is arranged at the air inlet 2, so that the mechanical grease separator 7 not only realizes the separation of grease from other gases, but also can effectively intercept impurities entering the air inlet channel, and flames of the cooking bench can be prevented from entering the air inlet channel 4; when the mechanical grease separator 7 is of a radiation type mesh disc structure, the mechanical grease separator 7 is arranged in the air inlet channel 4 and is combined with the fan device 6, namely, the mechanical grease separator 7 and a fan driving motor of the fan device 6 are concentric and coaxial, and under the driving of the fan driving motor, high-speed rotation is realized to separate grease from other gases.

The gas purification device 8 is one or different combinations of more than two of an electrostatic lampblack purifier, an HEPA (High Efficiency Particulate Air) high-efficiency filter screen, an activated carbon filter screen, a TiO2 photocatalyst filter screen and an ultraviolet lamp (UV lamp tube). Under the suction action of the fan device 6, mixed gas enters the gas purification device 8, the gas purification device 8 purifies smoke, peculiar smell and harmful gas (including smoke particles, germs and viruses, VOCs and the like) in the mixed gas, and the treated clean gas enters the cooling device 9 under the action of the fan device 6. The gas purification device 8 can be arranged at the air outlet 3 or in the air outlet channel 5, or can be arranged in the air inlet channel 4, but preferably the gas purification device 8 is arranged at the air outlet 3 or in the air outlet channel 5. The gas purification device 8 has the advantage that a trace amount of grease remaining in the mixed gas subjected to grease separation is purified more deeply through high-speed centrifugal rotation of a centrifugal fan of the fan device 6, so that no grease exists in the mixed gas entering the air purification device 8, and the purification effect of the gas purification device is prevented from being affected and polluted.

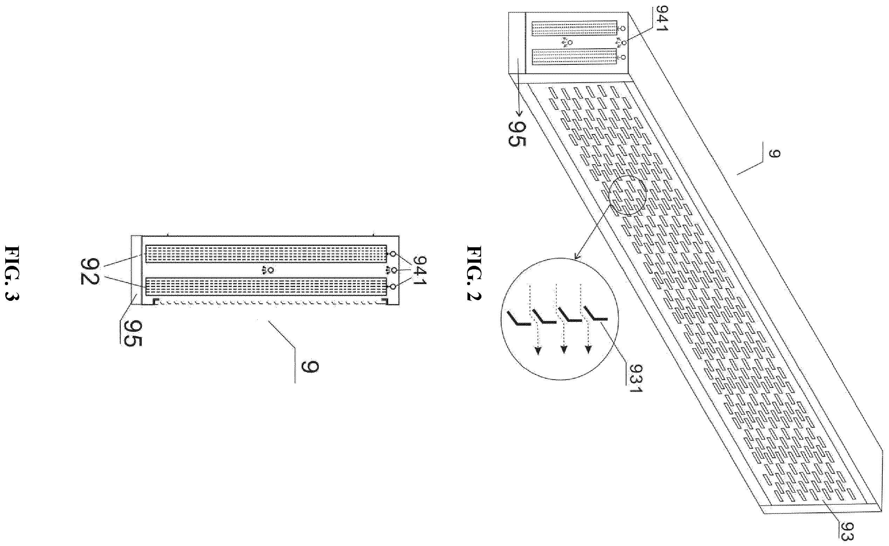

As shown in FIG. 2 and FIG. 3, the cooling device 9 comprises a water supply pipe 91, water spray guide plates 92, a water mist baffle plate 93, a spray pipe 94 and a water receiving tank 95, wherein the water supply pipe 91 communicates with an external water pipe, the spray pipe 94 communicates with the water supply pipe 91, spray nozzles 941 are arranged on the spray pipe 94 and face the water spray guide plates 92 to spray water drops or water mist, the water spray guide plates 92 are of transparent honeycomb net structures and uniformly distributed from up to down, the water receiving tank 95 is located below the water spray guide plates 92 and used for receiving flowing water, the water mist baffle plate 93 is provided with blades 931 at certain gaps, and the blades 931 are folded or bent inwards so as to intercept blown water drops, so that the water drops drip downwards along the blades towards the water receiving tank 95, and the water drops are prevented from scattering outside.

As an optional embodiment, the cooling device 9 further comprises a water storage tank and a water pump, and the water supply pipe 91 is connected with the water storage tank. The water pump pumps water in the water storage tank to supply the water to the water supply pipe 91, ice blocks can be placed in the water storage tank to reduce the water temperature in stuffy summer, and therefore the cooling effect of the cooling device 9 is improved. It is worthy remembering that the water storage tank and the water receiving tank 95 are combined into a whole, namely the water storage tank is the water receiving tank 95, so that the cooling device 9 can form an internal water circulation system, water recycling is facilitated, and due to the fact that part of water is lost in the using process, water only needs to be injected into the water storage tank (the water receiving tank 95) irregularly.

The cooling device 9 further comprises a drainage pipe 96, one end of the drainage pipe 96 communicates with the water receiving tank 95, and the other end of the drainage pipe 96 communicates with the outside of the internal circulation type lampblack purifier, and the drainage pipe 96 is used for discharging the water inside the water receiving tank 95.

When the spray nozzles 941 spray water (the temperature of the sprayed water is usually between 15 °C and 20 °C), the water spray guide plate 92 can form water curtains, so that the temperature of purified gas (the temperature of the purified gas is usually far higher than the indoor temperature and the purified gas contains high-temperature water vapor) is greatly reduced after passing through the water spray guide plates 92, and the influence of the high-temperature water vapor on the indoor environment is avoided, so that the finally discharged gas does not have obvious influence on the indoor temperature, meanwhile, high-temperature water vapor in the gas is eliminated, and the discomfort of indoor personnel is eliminated.

Preferred embodiments of the present disclosure have been described in detail above.

## Claims

1. An internal circulation type lampblack purifier, comprising a shell (1), an air inlet (2), an air outlet (3), an air inlet channel (4), an air outlet channel (5), a fan device (6), a mechanical grease separator (7), a gas purification device (8) and a cooling device (9), wherein: the air inlet (2) is arranged on the shell (1); one end of the air inlet channel (4) communicates with the air inlet (2), the other end of the air inlet channel (4) communicates with the fan device (6); the mechanical grease separator (7) is arranged at the air inlet (2) or in the air inlet channel (4); and the cooling device (9) comprises a water supply pipe (91), water spray guide plates (92), a spray pipe (94) and a water receiving tank (95), wherein: the spray pipe (94) communicates with the water supply pipe (91); spray nozzles (941) are arranged on the spray pipe (94) and face the water spray guide plates (92); the water receiving tank (95) is located below the water spray guide plates (92);
**characterized in that**,
the air outlet (3) is arranged on the shell (1), one end of the air outlet channel (5) communicates with the air outlet (3), and the other end of the air outlet channel (5) communicates with the fan device (6), and the cooling device (9) is arranged at the air outlet (3); and
the cooling device (9) also comprises a water mist baffle plate (93), wherein the water mist baffle plate (93) is provided with blades (931) at certain gaps, and the blades (931) are folded or bent inwards.

2. The internal circulation type lampblack purifier according to claim 1, wherein the water spray guide plate (92) is of a transparent honeycomb net structure.

3. The internal circulation type lampblack purifier according to claim 1, wherein the cooling device (9) further comprises a water storage tank and a water pump, and the water supply pipe (91) is connected with the water storage tank.

4. The internal circulation type lampblack purifier according to claim 3, wherein the water storage tank and the water receiving tank (95) are combined into a whole.

5. The internal circulation type lampblack purifier according to claim 1, wherein the cooling device (9) further comprises a drainage pipe (96), one end of the drainage pipe (96) communicates with the water receiving tank (95), and the other end of the drainage pipe (96) communicates with the outside.

6. The internal circulation type lampblack purifier according to claim 1, wherein the mechanical grease separator (7) is of a double-layer concave-convex oppositely buckled grease separation screen plate structure or a motor-driven radiation type centrifugal screen disc structure.

7. The internal circulation type lampblack purifier according to claim 1, wherein the gas purification device (8) is one or different combinations of more than two of an electrostatic lampblack purifier, an High Efficiency Particulate Air, HEPA, high-efficiency filter screen, an activated carbon filter screen, a TiO2 photocatalyst filter screen and an ultraviolet lamp.

## Patentansprüche

1. Lampenschwarzreiniger vom internen Zirkulationstyp, umfassend eine Hülle (1), einen Lufteinlass (2), einen Luftauslass (3), einen Lufteinlasskanal (4), einen Luftauslasskanal (5), eine Lüftervorrichtung (6), einen mechanischen Schmiermittelseparator (7), eine Gasreinigungsvorrichtung (8) und eine Kühlvorrichtung (9), wobei: der Lufteinlass (2) an der Hülle (1) angeordnet ist; ein Ende des Lufteinlasskanals (4) mit dem Lufteinlass (2) kommuniziert, das andere Ende des Lufteinlasskanals (4) mit der Lüftereinrichtung (6) kommuniziert; der mechanische Schmiermittelseparator (7) an dem Lufteinlass (2) oder in dem Lufteinlasskanal (4) angeordnet ist; und die Kühlvorrichtung (9) ein Wasserversorgungsrohr (91), Wassersprühführungsplatten (92), ein Sprührohr (94) und einen Wasseraufnahmetank (95) umfasst, wobei: das Sprührohr (94) mit dem Wasserversorgungsrohr (91) kommuniziert; Sprühdüsen (941) an dem Sprührohr (94) angeordnet sind und den Wassersprühführungsplatten (92) zugewandt sind; der Wasseraufnahmetank (95) sich unterhalb der Wassersprühführungsplatten (92) befindet;
**dadurch gekennzeichnet, dass**
der Luftauslass (3) an der Hülle (1) angeordnet ist, wobei ein Ende des Luftauslasskanals (5) mit dem Luftauslass (3) kommuniziert und das andere Ende des Luftauslasskanals (5) mit der Lüftervorrichtung (6) kommuniziert, und wobei die Kühlvorrichtung (9) an dem Luftauslass (3) angeordnet ist; und
wobei die Kühlvorrichtung (9) ferner eine Wassernebelprallplatte (93) umfasst, wobei die Wassernebelprallplatte (93) in bestimmten Abständen mit Lamellen (931) versehen ist, und wobei die Lamellen (931) nach innen gefaltet oder gebogen sind.

2. Lampenschwarzreiniger vom internen Zirkulationstyp nach Anspruch 1, wobei die Wassersprühführungsplatte (92) eine transparente Wabennetzstruktur aufweist.

3. Lampenschwarzreiniger vom internen Zirkulationstyp nach Anspruch 1, wobei die Kühlvorrichtung (9) ferner einen Wasserspeichertank und eine Wasserpumpe umfasst, und wobei das Wasserversorgungsrohr (91) mit dem Wasserspeichertank verbunden ist.

4. Lampenschwarzreiniger vom internen Zirkulationstyp nach Anspruch 3, wobei der Wasserspeichertank und der Wasseraufnahmetank (95) zu einem Ganzen kombiniert sind.

5. Lampenschwarzreiniger vom internen Zirkulationstyp nach Anspruch 1, wobei die Kühlvorrichtung (9) ferner ein Abflussrohr (96) umfasst, wobei ein Ende des Abflussrohrs (96) mit dem Wasseraufnahmetank (95) kommuniziert und das andere Ende des Abflussrohrs (96) mit dem Wasseraufnahmetank (95) mit dem Äußeren kommuniziert.

6. Lampenschwarzreiniger vom internen Zirkulationstyp nach Anspruch 1, wobei der mechanische Schmiermittelseparator (7) aus einer doppelschichtigen konkavkonvexen entgegengesetzt gewölbten Schmiermittelseparatorplattenstruktur oder einer motorbetriebenen Zentrifugalfilterscheibenstruktur vom Strahlungstyp besteht.

7. Lampenschwarzreiniger vom internen Zirkulationstyp nach Anspruch 1, wobei die Gasreinigungsvorrichtung (8) eine oder verschiedene Kombinationen von mehr als zwei von einem elektrostatischen Lampenschwarzreiniger, einem Hochleistungspartikelfilter, HEPA, Hochleistungsfiltersieb, einem Aktivkohlefiltersieb, einem TiO2-Photokatalysatorfiltersieb und einer UV-Lampe ist.

## Revendications

1. Purificateur de noir de fumée de type à circulation interne, comprenant une coque (1), une entrée d'air (2), une sortie d'air (3), un canal d'entrée d'air (4), un canal de sortie d'air (5), un dispositif de ventilateur ( 6), un séparateur de graisse mécanique (7), un dispositif de purification de gaz (8) et un dispositif de refroidissement (9), dans lequel : l'entrée d'air (2) est disposée sur la coque (1) ; une extrémité du canal d'entrée d'air (4) communique avec l'entrée d'air (2), l'autre extrémité du canal d'entrée d'air (4) communique avec le dispositif de ventilateur (6) ; le séparateur de graisse mécanique (7) est disposé au niveau de l'entrée d'air (2) ou dans le canal d'entrée d'air (4) ; et le dispositif de refroidissement (9) comprend un tuyau d'alimentation en eau (91), des plaques de guidage de pulvérisation d'eau (92), un tuyau de pulvérisation (94) et un réservoir de réception d'eau (95), dans lequel : le tuyau de pulvérisation (94) communique avec le tuyau d'alimentation en eau (91) ; des buses de pulvérisation (941) sont disposées sur le tuyau de pulvérisation (94) et font face aux plaques de guidage de pulvérisation d'eau (92) ; le réservoir de réception d'eau (95) est situé au-dessous des plaques de guidage de pulvérisation d'eau (92) ;
**caractérisé en ce que**,
la sortie d'air (3) est disposée sur la coque (1), une extrémité du canal de sortie d'air (5) communique avec la sortie d'air (3), et l'autre extrémité du canal de sortie d'air (5) communique avec le ventilateur le dispositif (6) , et le dispositif de refroidissement (9) est disposé au niveau de la sortie d'air (3) ; et
le dispositif de refroidissement (9) comprend également une plaque déflectrice pour brouillard d'eau (93), la plaque déflectrice pour brouillard d'eau (93) étant dotée de pales (931) au niveau de certains espaces, et les pales (931) sont pliées ou courbées vers l'intérieur.

2. Purificateur de noir de fumée de type à circulation interne selon la revendication 1, dans lequel la plaque de guidage de pulvérisation d'eau (92) est constituée d'une structure en filet en nid d'abeilles transparente.

3. Purificateur de noir de fumée de type à circulation interne selon la revendication 1, dans lequel le dispositif de refroidissement (9) comprend en outre un réservoir de stockage d'eau et une pompe à eau, et le tuyau d'alimentation en eau (91) est relié au réservoir de stockage d'eau.

4. Purificateur de noir de fumée de type à circulation interne selon la revendication 3, dans lequel le réservoir de stockage d'eau et le réservoir de réception d'eau (95) sont combinés en un tout.

5. Purificateur de noir de fumée de type à circulation interne selon la revendication 1, dans lequel le dispositif de refroidissement (9) comprend en outre un tuyau de drainage (96), une extrémité du tuyau de drainage (96) communique avec le réservoir de réception d'eau (95), et l'autre extrémité du tuyau de drainage (96) communique avec l'extérieur.

6. Purificateur de noir de fumée de type à circulation interne selon la revendication 1, dans lequel le séparateur de graisse mécanique (7) est constitué d'une structure de plaque de tamis de séparation de graisse à double couche concave-convexe courbée de manière opposée ou d'une structure de disque de tamis de séparation de graisse du type à rayonnement entraîné par moteur.

7. Purificateur de noir de fumée de type à circulation interne selon la revendication 1, dans lequel le dispositif de purification de gaz (8) est une ou différentes combinaisons de plus de deux parmi un purificateur de noir de fumée électrostatique, un filtre à air particulaire à haute efficacité, HEPA, un tamis filtrant à haute efficacité, un filtre activé . un écran filtrant à charbon, un écran filtrant photocatalyseur TiO2 et une lampe ultraviolette.
